# DEMANDE DE BREVET EUROPEEN

(11) **EP 2 572 784 A1**
(43) Date de publication de la demande: **27.03.2013**
(21) Numéro de dépôt: 12185342.8
(22) Date de dépôt: 21.09.2012
(51) Int. Cl.: B01J 38/68, C12N 11/14

(54) **Procédé de régénération d'un catalyseur enzymatique**

(30) Priorité: 22.09.2011 FR 1158470; 31.01.2012 FR 1250923
(71) Demandeur: Total Raffinage Marketing, 92800 Puteaux (FR)
(72) Inventeur: Thomas, Daniel, 60150 Villers sur Coudun (FR); Pulvin, Sylviane, 60200 Compiegne (FR); Hedhli, Lofti, 78590 Noisy le Roi (FR); Djelassi, Samuel, 10600 La Chapelle Saint Luc (FR)
(74) Mandataire: Hirsch & Associés

(57) **Abrégé**

Procédé de régénération d'un catalyseur enzymatique disposé dans un réacteur comprenant un support minéral à base d'oxyde métallique et au moins d'une enzyme, caractérisé en ce qu'il contient au moins une étape de décrochage des enzymes usées par solvatation par balayage du catalyseur au moyen d'au moins un surfactant ionique, et au moins une étape de raccrochage des enzymes actives par balayage du dit support épuré par au moins une solution d'enzymes actives, ces deux étapes se faisant in-situ au sein du réacteur.

## Description

La présente invention concerne un procédé de régénération d'un catalyseur enzymatique par décrochage/accrochage d'une enzyme sur un support, la dite enzyme étant utilisée accrochée sur son support dans une réaction catalytique.

De nos jours de plus en plus de procédés font intervenir la catalyse enzymatique. Ces procédés nécessitent dans la vaste majorité des cas la fixation de l'enzyme sur un support solide particulaire, soit pour des réactions en batch, c'est-à-dire par lot, soit pour des réactions en lit fixe de catalyseur. Cependant, la durée de vie des enzymes est limitée dans le temps et la régénération du catalyseur contenant l'enzyme, délicate comparée aux catalyseurs traditionnels. La régénération du catalyseur consiste à décrocher l'enzyme usée puis à accrocher une enzyme active sur le support. Cette opération est souvent compliquée, notamment à l'échelle industrielle où les quantités d'enzymes mises en jeu sont importantes et dont le coût est répercuté directement dans le prix des produits fabriqués. En effet, jusqu'à présent, la régénération du catalyseur à base d'enzymes nécessite de vider les réacteurs du dit catalyseur, de traiter le support du dit catalyseur pour éliminer l'enzyme usée par exemple par traitement chimique et/ou par calcination, puis de recharger le support dans le réacteur et enfin de raccrocher une nouvelle enzyme active sur celui-ci pour reformer le catalyseur actif. Un tel procédé est lourd et coûteux car il allonge très fortement le temps d'inactivité du réacteur.

La présente invention vise à remédier à ces inconvénients en proposant un procédé qui ne nécessite pas de décharger le catalyseur pour changer l'enzyme, ni de vider le réacteur qui le contient, ce qui réduit le temps d'inactivité du réacteur.

La présente invention a donc pour objet un procédé de régénération d'un catalyseur enzymatique disposé dans un réacteur comprenant un support minéral à base d'au moins un oxyde métallique et au moins d'une enzyme, **caractérisé en ce qu'il** comprend au moins une étape de décrochage des enzymes du support par solvatation par balayage du catalyseur au moyen d'au moins un surfactant ionique jusqu'à élimination des enzymes usées, et au moins une étape de raccrochage d'enzymes actives par balayage du dit support épuré par au moins une solution d'enzymes actives, ces deux étapes se faisant in-situ au sein du réacteur.

Ce procédé permet de gagner non seulement du temps par rapport aux opérations de manutentions et de traitement du support, mais aussi un gain financier découlant d'une meilleure optimisation de l'utilisation des catalyseurs enzymatiques. Il présente en outre l'avantage d'être applicable à tous types d'enzymes supportées et toutes applications utilisant des enzymes supportées dans des réactions par catalyse enzymatique.

On entend par enzyme, une molécule permettant d'abaisser l'énergie d'activation d'une réaction et d'accélérer les réactions chimiques se déroulant dans le milieu sans modifier l'équilibre formé. Elles présentent aussi l'avantage d'être utilisables à température ambiante.

L'étape de décrochage des enzymes comprend le balayage du catalyseur par une solution aqueuse de surfactant ionique dit amphiphile, ce surfactant étant choisi dans le groupe constitué par les sels d'alkyls sulfonates, les sels d'alkyls sulfates, les sels d'alkyls sulfosuccinates, les sels d'alkyls phosphates esters, les sels d'alkylbenzène sulfonates, des sels d'ammonium quaternaires pris seuls ou en mélange.

Les sels d'ammonium sont généralement choisis parmi les sels de formule N R₁R₂R₃R₄⁺ dans laquelle R₁, R₂, R₃ et R₄ sont des groupements alkyles, aryles, aralkyles ou cycloalkyles comprenant de un à 30 atomes de carbone. Parmi ceux-ci sont préférés les sels de tétraméthylammonium, de tétraéthylammonium, de tétrapropylammonium, de tétrabutylammonium, de tétrapentylammonium, de Tétra-hexylammonium, de benzyltriméthylammonium, benzyltriéthylammonium et d'hexamethionium. Les sels de phosphonium correspondent structurellement en tous points aux sels d'ammonium précédemment cités.

Au cours de l'étape de décrochage des enzymes, on mesurera la quantité d'enzymes entraînées dans l'effluent de sortie du réacteur en mesurant en continu ou en discontinu son absorbance à une longueur d'onde caractéristique de l'enzyme recherchée par spectrométrie UV. Typiquement, les longueurs d'ondes correspondant aux enzymes varient de 280 nm (nanomètres) à 420 nm. Dans le cas, où l'enzyme utilisée est l'hémoglobine, la longueur d'onde caractéristique est de 404nm. La quantité d'enzymes va diminuer dans l'effluent de sortie tout le long de l'étape de décrochage. La concentration en enzymes usées mesurée par absorbance à une longueur d'onde caractéristique de l'enzyme en spectrométrie UV diminue dans l'effluent de sortie pendant toute la durée du dit balayage. La fin de cette étape sera atteinte lorsque la mesure différentielle de l'absorbance UV entre l'effluent de sortie et l'effluent d'entrée dans le réacteur, devient nulle.

Parmi les surfactants ioniques, les sels de métaux alcalins et alcalino-terreux des alkyls sulfates sont préférés. Ils sont choisis parmi les sels d'alkyls sulfates dont chaque groupement alkyl comprend de 6 à 20 atomes de carbone dans une chaîne paraffinique linéaire ou ramifiée, la dite chaîne comprenant de préférence de 10 à 16 atomes de carbone.

De préférence, le sel d'alkyl sulfate est un sel de sodium du sulfate de lauryl, encore appelé sodium dodecylsulfate (SDS). Le ou les surfactants ioniques sont introduits dans le réacteur en mélange avec de l'eau, leur concentration variant préférentiellement de 1 à 50 g/L, préférablement de 1 à 20 g/L.

Le procédé de l'invention permet le décrochage d'enzymes faisant partie du groupe constitué par les six classes d'enzymes, c'est-à-dire les hydrolases, les transférases, les oxydoréductases, les isomérases, les lyases ou décarboxylases et les lycases.

Parmi ces enzymes, le procédé est particulièrement adapté au décrochage des oxydoréductases, particulièrement des hémoprotéines et plus particulièrement de l'hémoglobine.

Les supports permettant le décrochage et l'accrochage d'enzymes selon le procédé de l'invention, sont de préféence des supports minéraux amorphes ou cristallins à base d'oxydes métalliques choisis dans le groupe des matériaux cristallins, amorphes ou composites comprenant l'alumine, la silice, la zircone, l'oxyde de titane ou de tout matériau composite comprenant au moins un de ces matériaux, de surface spécifique variant de 200 à 1000 m²/g, de préférence à partir de silice et/ou d'alumine de surface spécifique variant de 200 à 600 m²/g.

Selon le procédé de l'invention, le raccrochage des enzymes après l'opération de décrochage décrite précédemment est obtenue par balayage du support épuré par une solution d'enzyme jusqu'à ce que la concentration en enzyme, c'est-à-dire son absorbance mesurée à une longueur d'onde caractéristique de l'enzyme recherchée par spectrométrie UV, augmente dans l'effluent de sortie, pour atteindre la même absorbance que dans l'effluent entrant. L'étape de raccrochage se fait soit immédiatement soit plus tard, avec le même type d'enzyme ou un type d'enzyme différent.

L'étape de raccrochage des enzymes est terminée lorsque la mesure différentielle de la concentration en enzymes, exprimée par son absorbance, mesurée dans l'effluent d'entrée et dans l'effluent de sortie de réacteur, devient nulle. En effet la concentration de la solution d'enzymes en sortie est identique à celle de la solution entrant dans le réacteur, c'est-à-dire qu'elles présentent une absorbance identique.

On ne sortirait pas du cadre de l'invention si plusieurs enzymes de nature différente mais compatibles entre elles étaient introduites sur le support, ces enzymes étant introduites ensemble ou séquentiellement.

Dans un mode préféré de l'invention, le procédé comprend entre l'étape de décrochage des enzymes usées et l'étape d'accrochage des enzymes actives, une étape de lavage du support dans le réacteur par de l'eau pour éliminer les enzymes usées et surtout le surfactant résiduel.

Pendant cette étape de lavage, on mesurera l'absorbance par spectrométrie UV à la longueur d'onde de du SDS (260-280 nm) parce qu'il est préférable d'enlever la totalité de SDS servant au décrochage, avant le raccrochage des enzymes. La mesure différentielle de l'absorbance entre l'effluent de sortie et l'effluent entrant restera élevée tant que du surfactant seul ou en mélange avec de l'enzyme usée sera détecté dans la solution de lavage en sortie du dit réacteur. De ce fait, l'étape de lavage prendra fin quand la mesure différentielle de l'absorbance entre les deux effluents deviendra nulle, que ce soit au niveau de l'absorbance UV du SDS ou de celle des enzymes décrochées.

Selon un mode de réalisation, la fin du lavage est obtenue quand l'absorbance à la longueur d'onde caractéristique de l'enzyme dans l'effluent de sortie du réacteur devient nulle.

Après l'étape de raccrochage, si on souhaite utiliser le catalyseur enzymatique régénéré dans un milieu organique, on pourra avantageusement faire circuler dans le réacteur un mélange eau/solvant contenant une concentration évolutive de 100 à 0% d'eau pour de 0 à 100% d'un solvant organique dans le dit mélange, entre le début et la fin du séchage, le séchage correspondant à une élimination complète de l'eau sur le support et éventuellement retirer le surplus d'enzymes non accrochées. Ce solvant correspondra en général aux solvants choisis comme milieu réactionnel comme par exemple les cétones, les esters et les éthers.

Un deuxième objet de l'invention est l'application du procédé de l'invention à tous les catalyseurs enzymatiques dans lesquels les enzymes sont accrochées au support par des liaisons de faible énergie telles que des liaisons Van der Waals, des liaisons électrostatiques ou des liaisons hydrogène.

Un troisième objet de l'invention est l'utilisation en catalyse enzymatique des catalyseurs régénérés selon le procédé décrit précédemment.

Les exemples et figures données ci-après visent à décrire l'invention dans certaines de ses formes particulières mais ne peuvent être considérées comme limitant la portée de celle-ci.

### Exemple 1

Dans cet exemple, on prépare un catalyseur enzymatique frais. On introduit dans un réacteur 8,4 g de silice Davicat^{®}SI 1700 (Merck), de surface spécifique de 320 m2/g), puis une solution d'hémoglobine non modifiée (commercialisée par Vapran) à 10 g/l dans une solution tampon à pH=5. Une pompe HPLC est réglée sur un débit de 1 ml/mn pour introduire la solution d'hémoglobine dans le réacteur et le chronomètre est déclenché au moment où la première goutte d'hémoglobine pénètre dans le réacteur.

Des prélèvements des effluents en entrée et en sortie du réacteur sont effectués régulièrement afin de mesurer la concentration en hémoglobine, pour déterminer par mesure différentielle entre l'effluent entrant et l'effluent sortant, la quantité d'hémoglobine adsorbée par la silice. On mesure ainsi les absorbances de l'effluent entrant et de l'effluent de sortie par spectrophotométrie UV au moyen d'un spectromètre UV UVIKON XS commercialisé par Socoman à 404 nm, longueur d'onde correspondant à celle de l'hémoglobine. On suit ainsi la différence d'absorbance entre les deux effluents en fonction du temps d'injection.

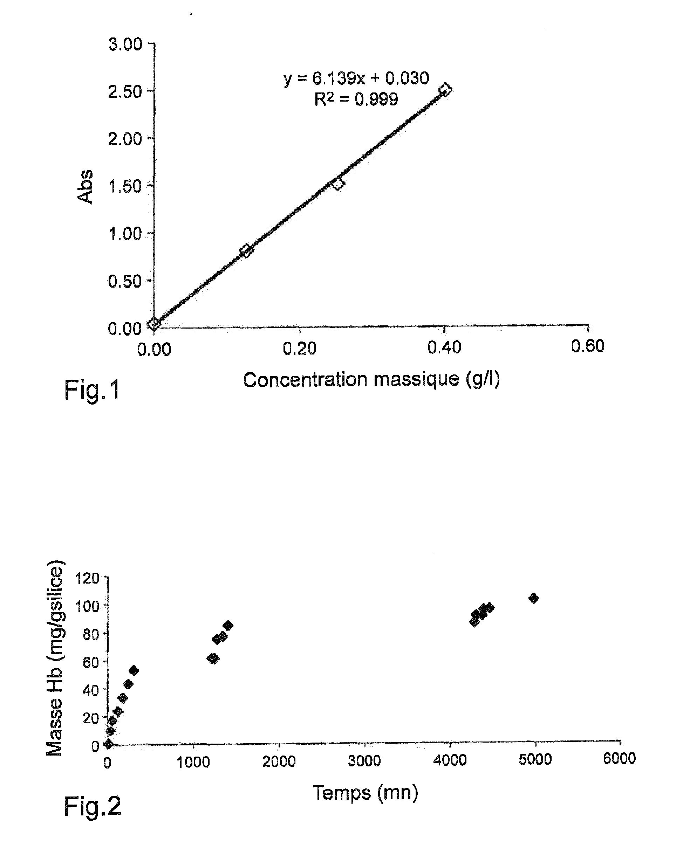

Bien entendu, le dit spectromètre UV a été préalablement étalonné, à partir de différentes solutions étalons d'hémoglobine dans l'eau à des concentrations différentes. La courbe étalon donnant la mesure d'absorbance UV en fonction de la concentration en hémoglobine de la solution d'hémoglobine utilisée par exemple pour l'accrochage (Voir Figure 1, la courbe d'étalonnage absorbance UV à 404 nm en fonction de la concentration de la solution d'hémoglobine).

En calculant la quantité d'hémoglobine introduite dans le réacteur avant que la mesure différentielle de l'absorbance entre l'entrée et la sortie ne devienne nulle, on peut déterminer la quantité maximale d'hémoglobine accrochée sur la silice. La Figure 2 montre l'évolution de la quantité d'hémoglobine, rapportée à la masse de silice introduite dans le réacteur, qui est accrochée sur la silice. La saturation de la silice en hémoglobine est ici de 100 mg/g de silice.

L'hémoglobine étant soluble dans l'eau jusqu'à une concentration de 100g/l, il est possible de répéter cette expérience à concentration plus forte (par exemple 30 ou 50 g/l) afin de réduire le temps nécessaire à l'accrochage de l'hémoglobine sur la silice.

### Exemple 2

Cet exemple décrit l'effet du débit d'entrée de la solution d'hémoglobine sur la vitesse d'accrochage de celle-ci sur le support en silice pour apprécier les performances en adsorption du support pour un catalyseur enzymatique frais.

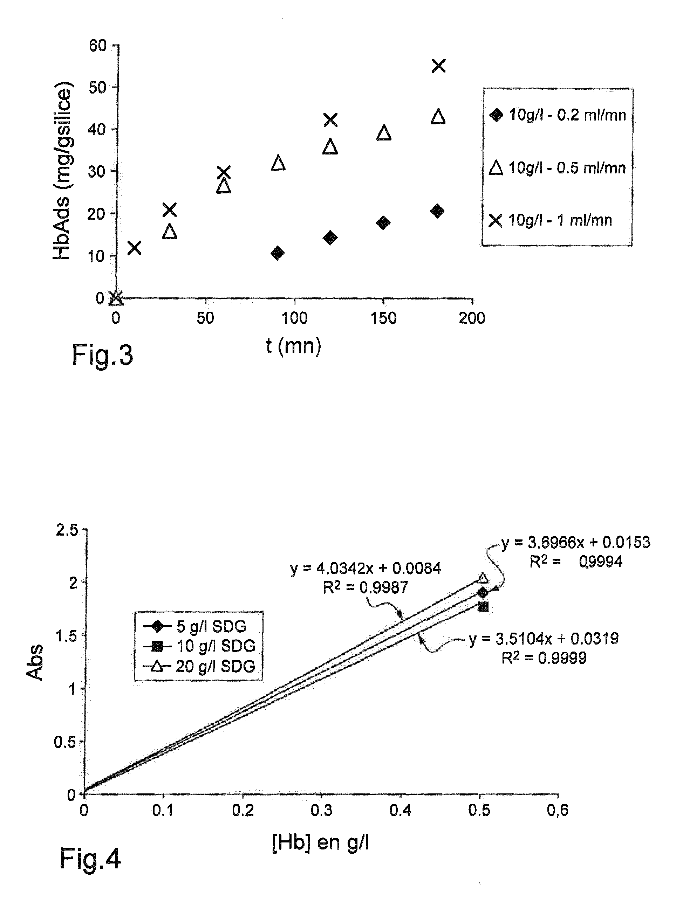

Comme dans l'exemple 1, on utilise comme effluent d'entrée une solution d'hémoglobine à 10g/l dans de l'eau qui est percolée dans le réacteur préalablement garni de 8,4 g de silice Davicat^{®}SI 1700. Le débit de la pompe HPLC est réglée pour varier de 0,2 à 1 ml/mn selon les expériences (dans la Figure 3 ; 0,2 ml/minute ; 0,5 ml/minute ; 1 ml/minute), et pour chacune d'elles, le chronomètre est déclenché au moment où la première goutte de solution d'hémoglobine pénètre dans le réacteur.

Des prélèvements de l'effluent en sortie du réacteur sont effectués régulièrement afin de suivre l'évolution de la concentration en hémoglobine, donc la quantité d'hémoglobine qui est restée accrochée sur le support en silice par rapport à la quantité d'hémoglobine dans l'effluent d'entrée du réacteur. Comme précédemment, la quantité d'hémoglobine adsorbée par la silice est obtenue à partir de la mesure différentielle entre les absorbances de l'effluent d'entrée toujours constant et celles des effluents de sortie en fonction de la vitesse d'injection de la solution d'hémoglobine.

La Figure 3 présente l'évolution de la quantité d'hémoglobine accrochée par gramme de silice, en fonction du débit de la pompe d'alimentation en solution d'hémoglobine. On constate que la vitesse d'adsorption de l'hémoglobine sur la silice est d'autant plus importante que le débit de l'effluent en entrée du réacteur est grand. L'augmentation de débit permet de réduire le temps de saturation de la silice en hémoglobine.

### Exemple 3

Le présent exemple décrit l'influence de la quantité de sodium lauryl sulfate ou SDS utilisé dans l'étape de décrochage de l'hémoglobine du support tel que préparé dans l'exemple 1 sur l'absorbance mesurée dans la solution d'hémoglobine, soit en entrée soit en sortie du réacteur.

Pour prendre en compte l'influence de la présence de SDS sur les mesures d'absorbance de la solution d'hémoglobine, nous avons effectué des mesures d'absorbance au spectromètre UV entre 350-550 nm de solutions d'hémoglobine contenant de teneurs en SDS variables, afin d'étalonner l'appareil.

Ces mesures ont été répétées pour deux concentrations en hémoglobine à 0,25g/l et à 50g/l.

Sur la Figure 4, quelle que soit la concentration d'hémoglobine dans l'eau dans l'effluent d'entrée, on constate une diminution de l'absorbance UV caractéristique de l'hémoglobine à 404nm en présence de SDS. Cependant, cette modification est stable dans le temps.

En effet, la concentration en SDS n'a que très peu d'influence sur l'absorbance de l'hémoglobine à 404 nm, et d'autre part, la linéarité est bien respectée quelque soit la concentration en SDS.

### Exemple 4

Le présent exemple décrit l'étape de décrochage de l'hémoglobine accrochée sur la silice dans le cas du catalyseur préparé dans l'exemple 2. Ainsi, on dispose d'un catalyseur enzymatique contenant 93 mg/g d'hémoglobine absorbés sur de la silice.

Au cours de l'étape de décrochage, une solution aqueuse de SDS à 10g/l est introduite dans le réacteur à un débit de 1 ml/mn via la pompe HPLC précédemment décrite.

La quantité d'hémoglobine décrochée de la silice est mesurée en comparant les valeurs de l'absorbance dans l'effluent d'entrée contenant que du SDS en solution et dans l'effluent de sortie contenant toujours du SDS mais aussi de l'hémoglobine.

L'étalonnage du spectromètre UV a été fait comme décrit dans l'exemple 1 avec différentes solutions étalons d'hémoglobine en présence de SDS. Les courbes étalons donnant la mesure d'absorbance correspondant à la concentration en hémoglobine de la solution étalon (voir Figure 4).

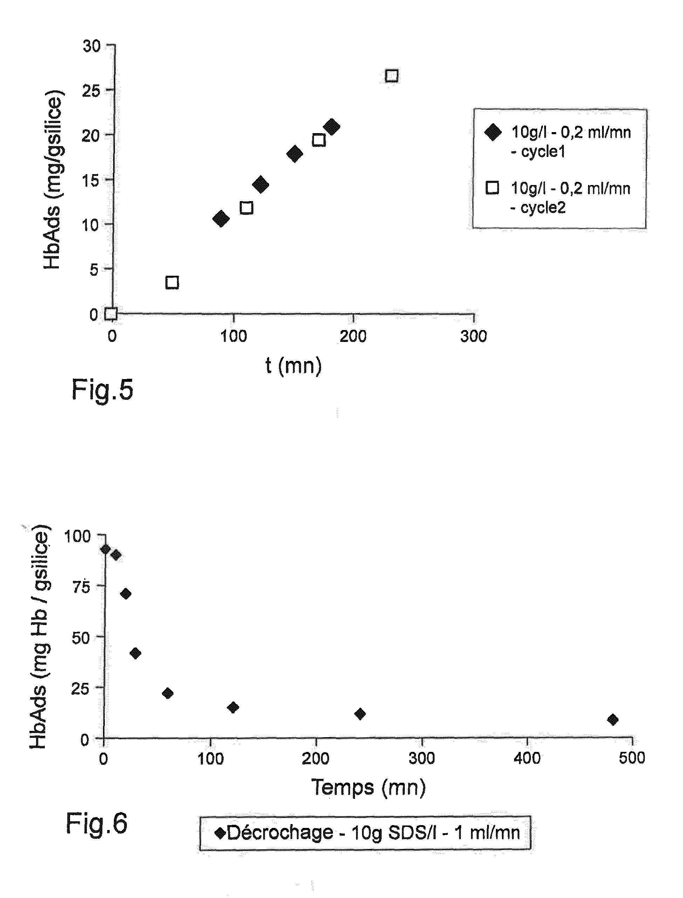

Des échantillons de l'effluent de sortie sont prélevés à 10mn, 20 mn, 30 mn, 60 mn, 120mn, 240mn et 480 mn pour évaluer leur absorbance à 404nm et donc leur concentration en hémoglobine en sortie du réacteur. Le profil de désorption de l'hémoglobine lors de la phase de décrochage a été représenté dans la Figure 6 à partir de ces valeurs mesurées.

A la fin de cette étape de décrochage, 88 mg d'hémoglobine/g de silice ont été décrochés et récupérés en sortie de réacteur, soit 95% de la quantité d'hémoglobine initialement absorbée sur la silice.

### Exemple 5

Le présent exemple décrit l'étape de raccrochage d'hémoglobine sur le support duquel on vient de décrocher l'hémoglobine selon l'exemple 4, après une étape de lavage ou de rinçage du réacteur à l'eau jusqu'à ce que plus de SDS soit détecté dans l'effluent de sortie du réacteur.

A la fin de cette étape, on réintroduit en continu comme décrit dans l'exemple 2 une solution d'hémoglobine à 10g/l jusqu'à ce les absorbances UV de l'effluent en entrée de réacteur et celle de l'effluent en sortie de réacteur soient identiques, la teneur en hémoglobine en entrée et en sortie étant égale.

L'évolution de la quantité d'hémoglobine fixée est donnée dans la Figure 5, montrant les deux cycles d'accrochage. Le cycle 1 correspond à l'accrochage tel que décrit dans l'exemple 2 et le cycle 2 au raccrochage effectué après les étapes de décrochage et de lavage du réacteur décrites dans le présent exemple et dans l'exemple 4.

On constate que les profils d'immobilisation correspondant du premier cycle d'accrochage pour préparer du catalyseur frais et du second cycle après décrochage de l'enzyme se superposent. Le traitement au SDS pour le décrochage de l'hémoglobine de la silice n'entraine donc pas de diminution du potentiel d'absorption de l'hémoglobine sur la silice, en vue de son immobilisation.

## Revendications

1. Procédé de régénération d'un catalyseur enzymatique disposé dans un réacteur comprenant un support minéral à base d'au moins un oxyde métallique et au moins d'une enzyme, **caractérisé en ce qu'il** contient au moins une étape de décrochage des enzymes par solvatation par balayage du catalyseur au moyen d'au moins un surfactant ionique, et au moins une étape de raccrochage d'enzymes actives par balayage du dit support épuré par au moins une solution d'enzymes actives, ces deux étapes se faisant in-situ au sein du réacteur.

2. Procédé selon la revendication 1, **caractérisé en ce que** l'étape de décrochage de l'enzyme comprend le balayage du catalyseur par une solution aqueuse de surfactant ionique dit amphiphile choisi dans le groupe constitué par les sels d'alkyls sulfonates, les sels d'alkyls sulfates, les sels d'alkyls sulfosuccinates, les sels d'alkyls phosphates esters, les sels d'alkylbenzène sulfonates, et les sels d'ammonium quaternaires.

3. Procédé selon l'une des revendications précédentes, **caractérisée en ce que** la concentration en enzymes usées mesurée par son absorbance à une longueur d'onde caractéristique de l'enzyme en spectrométrie UV diminue dans l'effluent de sortie pendant toute la durée du dit balayage.

4. Procédé selon l'une des revendications précédentes, **caractérisé en ce que** la fin de l'étape de décrochage est atteinte lorsque la mesure différentielle de la concentration en enzymes exprimée par son absorbance entre l'effluent de sortie et l'effluent d'entrée dans le réacteur, devient nulle.

5. Procédé selon l'une des revendications précédentes, **caractérisé en ce que** parmi les surfactants ioniques, les sels de métaux alcalins des alkyls sulfates sont choisis parmi les sels d'alkyls sulfate, chaque groupement alkyl comprenant de 6 à 20 atomes de carbone dans une chaine paraffinique linéaire ou ramifiée, de préférence de 10 à 16 atomes de carbone.

6. Procédé selon la revendication 5, **caractérisé en ce que** le sel d'alkyl sulfate est un sel de sodium du sulfate de lauryl.

7. Procédé selon l'une des revendications précédentes, **caractérisé en ce que** les enzymes décrochées par le dit procédé font partie des six classes d'enzymes, les hydrolases, les transférases, les oxydoréductases, les isomérases, lyases ou décarboxylases et les lycases.

8. Procédé selon l'une des revendications précédentes, **caractérisé en ce que** les enzymes décrochées font partie des oxydoréductases, particulièrement des hémoprotéines et l'enzyme plus particulièrement préférée est de l'hémoglobine.

9. Procédé selon l'une des revendications précédentes, **caractérisé en ce que** les étapes de décrochage et d'accrochage des enzymes sont faites sur des supports minéraux amorphes ou cristallins à base d'oxydes métalliques choisis dans le groupe des matériaux cristallins, amorphes ou composites comprenant l'alumine, la silice, la zircone, l'oxyde de titane ou tout matériau composite comprenant au moins un de ces matériaux, de surface spécifique variant de 200 à 1000 m²/g, de préférence à partir de silice et/ou d'alumine de surface spécifique variant de 200 à 600 m²/g.

10. Procédé selon l'une des revendications précédentes, **caractérisé en ce que** l'étape de raccrochage de l'enzyme est obtenue par balayage du support épuré par une solution d'enzymes jusqu'à ce que la concentration en enzymes, c'est-à-dire son absorbance à la longueur d'onde caractéristique, augmente dans l'effluent de sortie.

11. Procédé selon la revendication 10, **caractérisé en ce que** l'étape de raccrochage de l'enzyme est terminée lorsque la mesure différentielle de la concentration en enzymes, mesurée dans l'effluent d'entrée et dans l'effluent de sortie de réacteur, devient nulle.

12. Procédé selon l'une des revendications précédentes, **caractérisé en ce que** le procédé comprend entre l'étape de décrochage des enzymes usées et l'étape d'accrochage des enzymes actives, une étape de lavage du support dans le réacteur par de l'eau pour éliminer les enzymes usées et surtout le surfactant résiduel.

13. Procédé selon la revendication 12, **caractérisé en ce que** la fin du lavage est obtenue quand l'absorbance à la longueur d'onde caractéristique de l'enzyme dans l'effluent de sortie du réacteur devient nulle.

14. Application du procédé selon l'une des revendications précédentes, pour régénérer tous les catalyseurs enzymatiques dont l'enzyme est accrochée au support par des liaisons de faibles énergie, telles que des liaisons Van der Waals, des liaisons électrostatiques ou des liaisons hydrogène.

15. Utilisation des catalyseurs régénérés par le procédé selon l'une des revendications 1 à 13, dans la catalyse enzymatique.
